# EUROPÄISCHE PATENTSCHRIFT

(11) **EP 1 816 969 B1**
(45) Veröffentlichungstag und Bekanntmachung des Hinweises auf die Patenterteilung: **24.10.2012**
(21) Anmeldenummer: 05811039.6
(22) Anmeldetag: 07.11.2005
(51) Int. Cl.: A61B 18/12

(54) **HF-CHIRURGIEGERÄT**
HF SURGICAL INSTRUMENT
INSTRUMENT CHIRURGICAL HF

(30) Priorität: 11.11.2004 DE 102004054575
(43) Veröffentlichungstag der Anmeldung: 15.08.2007
(73) Patentinhaber: ERBE Elektromedizin GmbH, 72072 Tübingen (DE)
(72) Erfinder: FISCHER, Klaus, 72202 Nagold (DE); WERNER, Erich, 72827 Wannweil (DE)
(74) Vertreter: Bohnenberger, Johannes
(86) Internationale Anmeldenummer: PCT/EP2005/011891
(87) Internationale Veröffentlichungsnummer: WO 2006/050888

(56) Entgegenhaltungen:
- EP-A- 0 253 012
- WO-A-03/005918
- DE-A1- 3 530 335

## Beschreibung

Die Erfindung betrifft ein HF-Chirurgiegerät nach dem Oberbegriff des Patentanspruches 1.

Die Hochfrequenzchirurgie wird seit vielen Jahren sowohl in der Human- als auch in der Veterinärmedizin eingesetzt, um biologisches Gewebe zu koagulieren und/oder zu schneiden. Dabei wird mit Hilfe geeigneter elektrochirurgischer Instrumente hochfrequenter Strom durch das zu behandelnde Gewebe geleitet, so dass sich dieses aufgrund Eiweißkoagulation und Dehydratation verändert. Das Gewebe zieht sich dabei derart zusammen, dass die Gefäße verschlossen und Blutungen gestillt werden. Eine darauf folgende Erhöhung der Stromdichte bewirkt ein explosionsartiges Verdampfen der Gewebeflüssigkeit und ein Aufreißen der Zellmembranen, wobei das Gewebe vollständig durchtrennt wird. Verfahren dieser Art weisen gegenüber einem rein mechanisch vorgenommenen Schnitt den Vorteil einer Hämostase der Schnittränder auf.

Zur Durchführung einer Koagulation und/oder eines Schneidvorganges werden HF-Chirurgiegeräte verwendet, die u. a. einen HF-Generator zur Erzeugung einer hochfrequenten Spannung und damit des hochfrequenten Wechselstromes aufweisen, sowie Steuer- und Regeleinrichtung zum An- und Abschalten des HF-Generators bzw. zum Unterbrechen eines HF-Stromkreises. Weiterhin sind Ein- und Ausgangsanschlüsse zum Anschluss von externen Schaltern und verschiedenen elektrochirurgischen Instrumenten vorgesehen. Ein Chirurgiegerät zur Durchführung einer Koagulation ist beispielsweise aus der EP 0 253 012 A bekannt.

Nach einem Koagulationsvorgang und insbesondere nach einem Schneidvorgang soll der HF-Generator zu einem geeigneten Zeitpunkt abschalten bzw. der HF-Stromkreis unterbrochen werden, damit eine zu starke und auch unnötige Beeinträchtigung des behandelten Gewebes vermieden wird. Dazu ist es notwendig, die einzelnen Phasen eindeutig zu identifizieren, um den Stromkreis zu einem geeigneten Zeitpunkt zu unterbrechen. Da sich die elektrochirurgischen Vorgänge im Millisekunden-Bereich bewegen, wird durch ein manuelles Schalten der optimale Endzeitpunkt eines elektrochirurgischen Vorganges kaum getroffen. Insofern sehen bekannte HF-Chirurgiegeräte die oben genannte Steuerungseinrichtung vor, der z. B. ein Lichtbogenmonitor zugeordnet ist. Der Lichtbogenmonitor erkennt beispielsweise anhand auftretender höherer harmonischer Frequenzen oder auch anhand nichtharmonischer Frequenzen der treibenden Spannung bzw. des HF-Stromes, dass ein Lichtbogen zwischen der aktiven Elektrode/den aktiven Elektroden und dem Gewebe gezündet hat. Das Kriterium der Lichtbogenerkennung dient dazu, einen einsetzenden Schneidvorgang zu detektieren. Um den mittels des Lichtbogens erkannten Schneidvorgang nun fortzuführen, wird dieser oftmals über einen Zeitgeber für eine vorgegebene Zeitspanne aufrechterhalten. Je nach Randbedingungen, die den Operationsverlauf beeinflussen, wie z. B. Gewebebedingungen oder auch die Handhabung des elektrochirurgischen Instruments, lässt sich jedoch der Übergang, also der exakte Schneidbeginn, nicht genau festlegen, weil der Lichtbogen nur verzögert erkannt wird.

Aus der DE 35 30 335 C2 ist ein HF-Chirurgiegerät zum Schneiden und Koagulieren bekannt, das u. a. eine Lichtbogenmonitoreinrichtung der oben beschriebenen Art, beispielsweise zum Steuern einer Schneidphase, aufweist. Der Schneidvorgang erfolgt hier in Zeitintervallen, deren Dauer einstellbar ist und deren Beginn durch einen Lichtbogen getriggert wird. Das Beenden einer einzelnen Schneidphase erfolgt jeweils nach Ablauf eines Zeitintervalls. Demgemäß werden einzelne Schneidimpulse bei einem sogenannten fraktionierten Schneiden mittels des Lichtbogenmonitors in Verbindung mit einem Zeitgeber gesteuert, wobei oben genannte Probleme auftreten. Es ist nicht gewährleistet, dass der Lichtbogen bei einem ersten Auftreten sofort erkannt wird. So kann ein Zeitfenster für einen Schneidimpuls beispielsweise zu groß sein und es wird zu weit geschnitten. Fällt der Schneidimpuls jedoch zu kurz aus, kommt es ggf. nicht zu einer Schneidwirkung und das Gewebe wird bestenfalls koaguliert. Auch hier werden also Randbedingungen, die den Operationsverlauf beeinflussen, wie z. B. Gewebebedingungen oder auch die Handhabung des elektrochirurgischen Instruments, nicht berücksichtigt, die Schneidphase wird nämlich unabhängig von äußeren Umständen und unabhängig von einem Schneidvorgang ausschließlich aufgrund des Ablaufs der definierten Zeitspanne beendet.

Der Erfindung liegt daher die Aufgabe zu Grunde, ein HF-Chirurgiegerät der eingangs genannten Art dahin gehend weiterzubilden, dass eine Schneidleistung verbesserbar ist.

Diese Aufgabe wird durch ein HF-Chirurgiegerät nach Patentanspruch 1 gelöst.

Insbesondere wird die Aufgabe durch ein HF-Chirurgiegerät zum Behandeln, insbesondere zum Schneiden und Koagulieren biologischen Gewebes mittels eines HF-Stromes, umfassend einen HF-Generator zum Zuführen eines HF-Stromes zu einer Schneidelektrode, mindestens eine Steuerungseinrichtung zum Unterbrechen eines HF-Stromkreises, wobei die Steuerungseinrichtung eine Lichtbogenmonitoreinrichtung zur Erkennung eines Lichtbogens und eine Strommonitoreinrichtung zur Erfassung des HF-Stromes umfasst, wobei die Strommonitoreinrichtung derart ausgebildet ist, dass sie die Amplitude des HF-Stromes erfasst und ein erstes Abschaltsignal dann erzeugt, wenn als ein erstes Abschaltkriterium der HF-Strom über eine definierte Zeitspanne abfällt und/oder der HF-Strom einen Zustand des behandelten Gewebes kennzeichnenden Schwellenwert unterschreitet, die Lichtbogenmonitoreinrichtung derart ausgebildet ist, dass sie ein zweites Abschaltsignal dann erzeugt, wenn als ein zweites Abschaltkriterium ein Lichtbogen zwischen der Schneidelektrode und dem Gewebe erkennbar entsteht, wobei die Steuerungseinrichtung zur Erfassung einer Schneidwirkung während einer Dampfphase derart ausgebildet ist, dass ein Erreichen des ersten Abschaltkriteriums überprüft wird und bei Nicht-Erreichen des ersten Abschaltkriteriums das zweite Abschaltkriterium überprüft wird, so dass auf das erste Abschaltsignal oder das zweite Abschaltsignal hin der HF-Stromkreis unterbrochen wird. Das Chirurgiegerät zeichnet sich dadurch aus, dass der Strommonitoreinrichtung eine zweite Zeitgebereinrichtung zugeordnet ist, die einen Schneidmodus nach dem Erzeugen des ersten Abschaltsignals für eine definierte Zeitspanne aktiv hält, und dass der Lichtbogenmonitoreinrichtung eine dritte Zeitgebereinrichtung zugeordnet ist, die den Schneidmodus nach dem Erkennen des Lichtbogens für eine definierte Zeitspanne aktiv hält.

Ein wesentlicher Punkt der Erfindung liegt darin, dass ein tatsächlicher Beginn eines Schneidvorganges erfasst wird und damit jegliche Schneidwirkung Berücksichtigung findet. Durch die zweite Zeitgebereinrichtung kann der Schneidmodus selbsttätig für eine gewünschte Zeitspanne aufrechterhalten werden, ohne dass der Chirurg in den Ablauf eingreifen muss. Die Zeitspanne kann z. B. bereits vor dem Eingriff festgelegt werden. Selbstverständlich ist es möglich, den Schneidmodus bereits mit Erzeugung des Abschaltsignals zu unterbrechen. Da erfindungsgemäß die Lichtbogenmonitoreinrichtung nur dann aktiv wird, wenn die Strommonitoreinrichtung den HF-Stromkreis nicht vorab unterbricht und die Erkennung des Lichtbogens einen eindeutigen Hinweis auf den Beginn einer Schneidphase gibt, kann mittels der dritten Zeitgebereinrichtung auf einfache Weise eine beliebig definierte Schnittlänge vorgegeben werden, die dann einer wahren Schnittlänge entspricht.

Innerhalb eines Schneidmodus ist die Spannung nämlich ausreichend hoch, so dass bei ausreichend starker Koagulation des Gewebes und einer damit verbundenen einsetzenden Dampfphase bereits zu Beginn der Dampfphase die Möglichkeit der Lichtbogenbildung besteht. Der Schneidprozess beginnt also mit Eintritt der Dampfphase, wobei ein Lichtbogen, beispielsweise aufgrund Signalrauschens, noch nicht erkannt werden kann. Je nach Gewebestruktur, Ausgestaltung des elektrochirurgischen Instruments, dessen Handhabung und anderer die Operation beeinflussender Randbedingungen unterscheiden sich die Dampfphasen insbesondere hinsichtlich ihrer Länge voneinander. Bei sehr kurzen Dampfphasen ist die verzögerte Erkennung unerheblich; mit dem erkennbaren Auftreten eines Lichtbogens wäre eine hinreichend genaue Erfassung des Beginns einer Schneidphase gewährleistet. Bei sehr langen Dampfphasen kann die dort bereits auftretende Schneidwirkung jedoch nicht unberücksichtigt bleiben. Insofern wird hier ein Strommonitor für das rechtzeitige Erkennen der Schneidphase eingesetzt.

Dies bedeutet, dass nun aufgrund der Steuerungseinrichtung letztendlich - gemäß dieser Erfindung - entweder über den Strommonitor oder den Lichtbogenmonitor die Schneidwirkung erfassbar ist. Entweder wird also ein gegebenenfalls auftretender erkennbarer Lichtbogen erfasst oder aber ein charakteristischer Stromabfall. Zur Erfassung des Stromabfalls wird der Stromverlauf über die definierte Zeitspanne verfolgt, idealerweise über die Erfassung der Amplitude bzw. des Amplitudenverlaufs, derart, dass der wahre Stromabfall gegenüber lokaler Maxima und Minima hervortritt und erkennbar wird. So kann zur Erfassung des Stromabfalls beispielsweise die gemittelte Stromstärke innerhalb des hinreichend großen Zeitintervalls betrachtet werden (gleitender Mittelwert), um die Differenz zwischen lokalen Maxima und Minima zu verringern. Die Mittelwertbildung verringert die Wahrscheinlichkeit einer fehlerhaften Unterbrechung des HF-Stromkreises durch die Strommonitoreinrichtung aufgrund einer etwaigen falschen Interpretation des Kurvenverlaufs. Die Verringerung der Differenz zwischen den lokalen Maxima und Minima ermöglicht ebenfalls eine genauere Erkennung des gegebenenfalls zu erreichenden Schwellenwertes.

In einer ersten bevorzugten Ausführungsform übermittelt die Steuerungseinrichtung das erste Abschaltsignal oder das zweite Abschaltsignal an den HF-Generator, so dass dieser abschaltet und den HF-Stromkreis unterbricht. Damit wird eine besonders einfache und zuverlässige Ausgestaltung zur Unterbrechung des Stromkreises realisiert.

In einer weiteren bevorzugten Ausführungsform ist mindestens eine erste Signalverarbeitungseinrichtung vorgesehen, an die das erste Abschaltsignal oder das zweite Abschaltsignal zuführbar ist, wobei die erste Signalverarbeitungseinrichtung das jeweilige Abschaltsignal als Einschaltsignal an den HF-Generator übermittelt, so dass dieser einschaltet und der HF-Stromkreis geschlossen wird. Da mit dem Abschalten des HF-Generators und demgemäß mit dem Unterbrechen des HF-Stromkreises gleichzeitig ein Schneidimpuls beendet wird, kann das Abschaltsignal genutzt werden, einen nächsten Schneidimpuls einzuleiten. Das heißt, das Abschaltsignal wird als Einschaltsignal über die erste Signalverarbeitungseinrichtung wieder dem HF-Generator zugeführt, so dass dieser erneut einschaltet. Dies ist insbesondere bei einem fraktionierten Schneiden, d. h. innerhalb eines intermittierenden Schneidmodus von Vorteil, bei dem erst durch die Abfolge mehrerer Schneidimpulse eine vollständige Gewebedurchtrennung erreicht wird. Damit kann sich der Operateur auf den Eingriff konzentrieren, während die Steuerung der Schneidphase selbsttätig abläuft.

Eine weitere bevorzugte Ausführungsform sieht vor, dass der mindestens ersten Signalverarbeitungseinrichtung mindestens eine erste Zeitgebereinrichtung zugeordnet ist, so dass die erste Signalverarbeitungseinrichtung den HF-Generator derart ansteuert, dass das Einschalten des HF-Generators nach einer definierten Zeitspanne erfolgt. Damit können beliebig lange Pausenintervalle zwischen den einzelnen Schneidimpulsen vorgesehen werden, um beispielsweise wiederholt eine Abkühlung des Operationsgebietes zu gewährleisten.

In einer bevorzugten Ausführungsform ist der Strommonitoreinrichtung eine Auswerteeinrichtung zugeordnet, die die Amplitude des HF-Stromes bzw. ganz allgemein den Stromverlauf durch Berechnen des Mittelwertes über eine definierte Anzahl jeweils zuletzt eingelesener Messwerte erfasst. Das heißt, es erfolgt eine permanente Erfassung von Amplitudenwerten des HF-Stromes bzw. ganz allgemein von Stromwerten und eine permanente Mittelwertbildung (gleitender Mittelwert). Damit ist entweder der Schwellenwert oder ein Abfall des HF-Stromes über eine definierte Zeitspanne zu ermitteln. Die Mittelwertbildung verringert die Wahrscheinlichkeit einer fehlerhaften Unterbrechung des HF-Stromkreises durch die Strommonitoreinrichtung aufgrund einer falschen Interpretation eines Kurvenverlaufs. Das heißt, es wird insbesondere vermieden, lokale Maxima oder Minima, also ein Rauschen, falsch auszulegen.

Eine erfindungsgemäße Lösung sieht vor, dass der Lichtbogenmonitoreinrichtung eine Detektionseinrichtung zugeordnet ist, die höhere harmonische Frequenzen und/oder nicht-harmonische Frequenzen des HF-Stromes als eine charakteristische Frequenz für ein Vorhandensein des Lichtbogens detektiert. Da ein Lichtbogen als ein nicht-linearer Widerstand wirkt, wird ein durch den Lichtbogen fließender Wechselstrom derart verzerrt, dass höhere harmonische oder auch nicht-harmonische Frequenzen des HF-Stromes gebildet werden. Durch das Erfassen dieser Frequenzen wird auf einfachste Art die Erkennung eines Lichtbogens bewerkstelligt.

Alternativ ist es möglich, eine Einrichtung vorzusehen, über die der Lichtbogen optisch erkannt und ein entsprechendes Signal generiert wird. Damit lässt sich der Lichtbogen auf einfache Weise identifizieren.

Eine vorteilhafte Realisierung der Vorrichtung besteht darin, mindestens eine zweite Signalverarbeitungseinrichtung vorzusehen, an die das erste Abschaltsignal oder das zweite Abschaltsignal zuführbar ist, wobei die zweite Signalverarbeitungseinrichtung mittels des ersten Abschaltsignals oder des zweiten Abschaltsignals eine optische und/oder akustische Anzeige derart ansteuert, dass die Unterbrechung des HF-Stromkreises aufgrund des ersten Abschaltsignals oder des zweiten Abschaltsignals zur Benutzerführung angezeigt wird. Mittels der Anzeige ist es möglich, während des weiteren Eingriffs einen bisher aufgezeichneten Schneidverlauf, d. h. bisher aufgenommene Randbedingungen, nachvollziehen zu können und damit sowohl Gewebestrukturen zu beurteilen als auch die weitere Handhabung des elektrochirurgischen Instruments darauf abzustimmen.

In einer bevorzugten Ausführungsform ist mindestens eine Speichereinrichtung vorgesehen, die die jeweils generierten Abschaltsignale innerhalb eines Eingriffs zum späteren und/oder gleichzeitigen Anzeigen eines Schneidverlaufes speichert. Damit können frühere Schneidverläufe abgerufen und daraus resultierende Erfahrungen für weitere Eingriffe genutzt werden.

Eine weitere bevorzugte Ausführungsform sieht vor, dass die Schneidelektrode als eine Schlingenelektrode ausgebildet ist. Schlingenelektroden für ein monopolares Schneiden eignen sich insbesondere zum Abtragen von Polypen oder sonstiger erhabener Gewebeteile, weil diese mittels der Schlinge ggf. ergriffen und gehalten werden können. Alternativ ist es möglich, bipolare Schlingenelektroden einzusetzen.

Weitere Ausführungsformen der Erfindung ergeben sich aus den Unteransprüchen.

Nachfolgend wird die Erfindung anhand von Ausführungsbeispielen beschrieben, die anhand der Abbildungen näher erläutert werden. Hierbei zeigen
- Fig. 1 ein funktionales Blockschaltbild, das eine Ausführungsform des erfindungsgemäßen HF-Chirurgiegerätes darstellt;
- Fig. 2 ein Strom-Zeit-Diagramm, das den Stromverlauf in einem Schneidmodus bei einer lose angelegten Schlinge zeigt;
- Fig. 3 ein Strom-Zeit-Diagramm, das den Stromverlauf in einem Schneidmodus bei einer fest angelegten Schlingen zeigt;
- Fig. 4 ein Flussdiagramm, das eine Arbeitsweise des HF-Chirurgiegerätes gem. Fig. 1 zeigt, wobei nur wesentliche Komponenten der erfindungsgemäßen Vorrichtung einbezogen sind.

In der nachfolgenden Beschreibung werden für gleiche und gleich wirkende Teile dieselben Bezugsziffern verwendet.

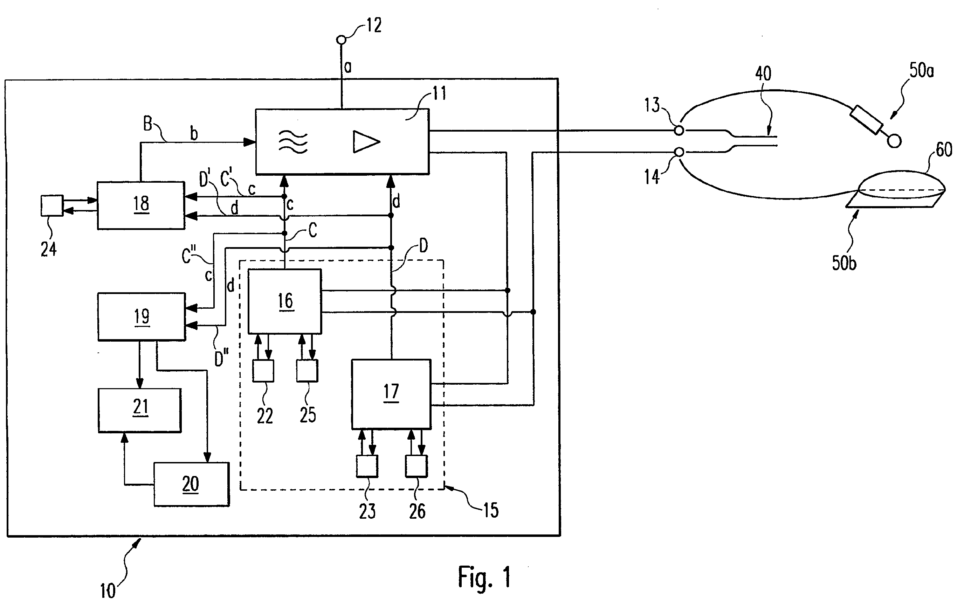

Fig. 1 zeigt eine Ausführungsform der erfindungsgemäßen Vorrichtung. Dabei sind schematisch die für die Erläuterung der Erfindung wesentlichen Komponenten eines HF-Chirurgiegerätes 10 sowie weitere Komponenten einer HF-Chirurgie-Anordnung gezeigt.

Das HF-Chirurgiegerät 10 weist einen Eingangsanschluss 12 zum Anschließen von Finger- und/oder Fußschalter aufweisenden Schalteinrichtungen (nicht gezeigt) auf. Über diese Schalteinrichtungen wird ein Aktivieren und/oder Deaktivieren des HF-Stromes ermöglicht. Die Schalteinrichtungen lassen sich hier vorzugsweise über eine Computeranordnung realisieren. Ausgangsseitig sind an dem HF-Chirurgiegerät 10 ein erster Ausgangsanschluss 13 und ein zweiter Ausgangsanschluss 14 vorgesehen, über die ein bipolares elektrochirurgisches Instrument 40 oder ein monopolares elektrochirurgisches Instrument 50a mit einer dazugehörigen Neutralelektrode 50b anschließbar ist. Diese Darstellung ist vereinfacht. Bei der praktischen Ausführung eines HF-Chirurgiegerätes sind zumeist unterschiedliche Anschlüsse für mono- oder bipolare Elektrodenanordnungen vorgesehen. Auch die Neutralelektrode ist schematisch dargestellt und bedeckt in der praktischen Anwendung vollständig einen Körperabschnitt 60 eines Patienten.

Kernstück des HF-Chirurgiegerätes 10 ist ein steuerbarer HF-Generator 11 zum Erzeugen eines HF-Stromes, genauer gesagt, zum Erzeugen einer Spannung. Über die Einstellung der Spannung lassen sich die gewünschten Stromstärken I_{HF} festlegen. Der HF-Generator 11 ist mit einer Steuerungseinrichtung 15 verbunden, wobei die Steuerungseinrichtung 15 eine Strommonitoreinrichtung 16 und eine Lichtbogenmonitoreinrichtung 17 aufweist. Die Steuerungseinrichtung 15 ist in eine Rückleitung von dem elektrochirurgischen Instrument zu dem HF-Generator 11 geschaltet. Der Strommonitoreinrichtung 16 ist eine Auswerteeinrichtung 22 und eine zweite Zeitgebereinrichtung 25 zugeordnet, während der Lichtbogenmonitoreinrichtung 17 eine Detektionseinrichtung 23 und eine dritte Zeitgebereinrichtung 26 zugeordnet ist.

In diesem Ausführungsbeispiel sind eine erste Signalverarbeitungseinrichtung 18 mit einer zugeordneten ersten Zeitgebereinrichtung 24 und eine zweite Signalverarbeitungseinrichtung 19 mit einer zugeordneten Anzeige 21 vorgesehen. Eine Speichereinrichtung 20 ist mit der Anzeige 21 und der zweiten Signalverarbeitungseinrichtung 19 verbunden.

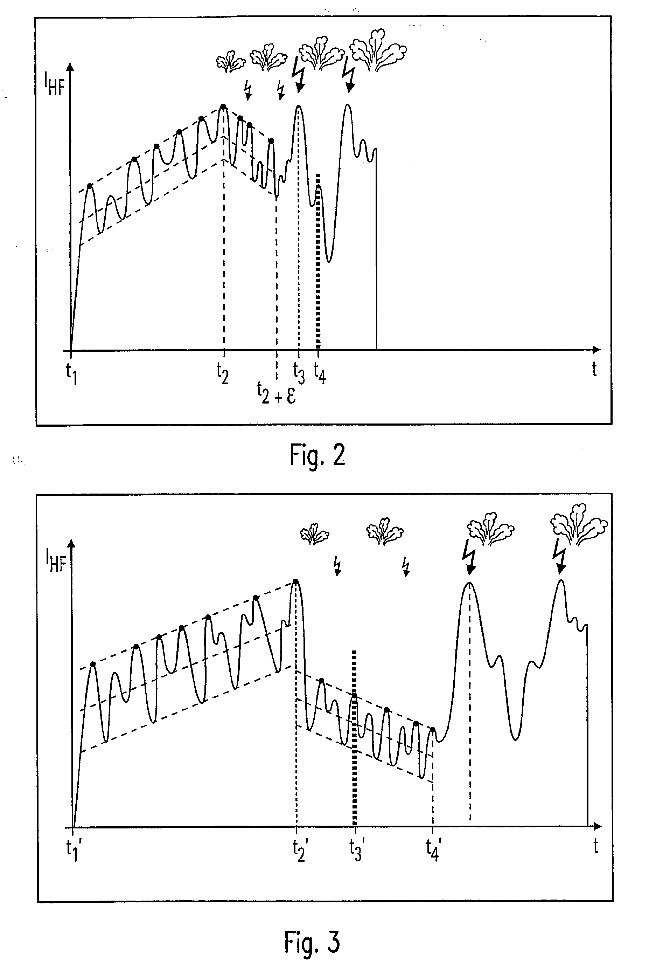

Die Wirkungsweise der Vorrichtung wird nachfolgend anhand der Fig. 2 und 3 beschrieben. Dabei wird beispielhaft ein endoskopisches Abtrennen eines Polypen mittels der monopolaren Schlingenelektrode 50a erläutert. Fig. 2 zeigt dabei ein Strom-Zeit-Diagramm. Bei diesem Stromverlauf ist die Schlinge 50a lose um den Polypen gelegt, weil die Gefahr eines Herausrutschens des Polypen nicht gegeben ist. Auch aus Fig. 3 ist ein Strom-Zeit-Diagramm zu entnehmen, wobei ein Stromverlauf beim Abtrennen des Polypen mit festgezogener Schlinge 50a wiedergegeben ist. Das Strangulieren des Polypen ist dann notwendig, wenn dieser aufgrund seiner Ausgestaltung leicht aus der Schlinge 50a herausrutschen könnte.

Durch Betätigung der Schalteinrichtungen liegt ein Einschaltsignal a an dem HF-Generator 11 an, der HF-Generator 11 ist in Betrieb und eine Schneidphase mittels der Schlingenelektrode 50a kann beginnen. In der praktischen Anwendung ist meist ein zusätzliches Einschaltkriterium notwendig, um Elektroden an dem elektrochirurgischen Instrument zu aktivieren. Dies kann z. B. mittels eines weiteren Handschalters an dem Instrument geschehen. Der HF-Strom wird über die Elektrode an das zu behandelnde Gewebe, d. h. an den Polypen, geleitet. Da die Steuerungseinrichtung 15, d. h. die Strommonitoreinrichtung 16 und die Lichtbogenmonitoreinrichtung 17 in die Rückleitung zu dem HF-Generator 11 geschaltet sind, registrieren diese einen Amplitudenverlauf des HF-Stromes bzw. eine Entstehung eines Lichtbogens.

Gemäß Fig. 2 wird der Schneidmodus zu einem Zeitpunkt t1 eingeschaltet, der Strom beginnt über die lose an den Polypen angelegte Schlingenelektrode 50a durch das zu behandelnde Gewebe zu fließen. Aufgrund der Erwärmung des Gewebes steigt die Stromstärke I_{HF} bis zu einem Zeitpunkt t2 an. Ab dem Zeitpunkt t2 beginnt das Gewebe so stark zu koagulieren, dass eine Dampfphase einsetzt. Aufgrund der durch den HF-Strom bedingten Wärmeentwicklung wird ein definierter Gewebebereich durch Eiweißkoagulation und Dehydratation verändert bzw. zerstört. Die im Solzustand vorliegenden kolloiden Gewebebestandteile gehen dabei zuerst in den Gelzustand über, wobei sich die nun gelförmigen Gewebebestandteile anschließend unter Austritt von Flüssigkeit weiter verdichten; das Gewebe verkocht. Der Widerstand des Gewebes steigt demgemäß an, so dass die Stromstärke I_{HF} aufgrund der sinkenden Leitfähigkeit des Gewebes bis zu einem Zeitpunkt t2 + ε abnimmt. Die Dampfphase zwischen t2 und t2 + ε ist in diesem Falle jedoch äußerst kurz ausgebildet, so dass ein Schneidvorgang während dieser Dampfphase kaum zu Buche schlägt. Aufgrund der sich nun immer weiter ausbildenden Isolierschicht an dem Gewebe zündet zu einem Zeitpunkt t3 ein nun erkennbarer Lichtbogen. Nach Erkennung des Lichtbogens durch die Lichtbogenmonitoreinrichtung 17 erzeugt diese ein Abschaltsignal d. Das Abschaltsignal d wird in diesem Ausführungsbeispiel an den HF-Generator 11 mittels einer Steuerleitung D übermittelt, so dass dieser zu einem gewünschten Zeitpunkt, z.B. zum Zeitpunkt t4, den HF-Stromkreis unterbricht, indem er beispielsweise abschaltet. Damit ist der Schneidvorgang beendet.

Wie Fig. 3 zu entnehmen ist, erwärmt sich ab einem Zeitpunkt t1' auch bei stranguliertem Polypen das zu behandelnde Gewebe bis zum Beginn einer starken Koagulation zu einem Zeitpunkt t2'. In diesem Falle ist ein charakteristischer Stromabfall zum Zeitpunkt t2' - dem Beginn einer Dampfphase - zu verzeichnen. Bereits zu diesem Zeitpunkt zünden aufgrund einer hohen Spannung im Schneidmodus Lichtbogen, die von der Lichtbogenmonitoreinrichtung 17 - wie bereits oben beschrieben - nicht erkannt werden. Die Strommonitoreinrichtung 16 erkennt jedoch den Stromabfall. Ein Abschaltkriterium für die Strommonitoreinrichtung 16 ist demgemäß z.B. ein abfallender HF-Strom-Mittelwert über eine festgelegte Zeitspanne t2' - t3'.

Alternativ ist es möglich, statt einer definierten Zeitspanne einen einen Zustand des behandelten Gewebes kennzeichnenden Schwellenwert durch die Strommonitoreinrichtung zu erfassen, so dass diese ein Abschaltsignal c erzeugt, sobald der Schwellenwert beispielsweise bei t3' erreicht wird. Das Abschaltsignal c wird in beiden Fällen mittels einer Steuerleitung C an den HF-Generator 11 übermittelt, so dass dieser zu einem gewünschten Zeitpunkt den HF-Stromkreis unterbricht, indem er beispielsweise abschaltet. Damit ist der Schneidvorgang zum Zeitpunkt t3' bereits vor Beendigung der Dampfphase zu einem Zeitpunkt t4' beendet.

Es lässt sich feststellen, dass die Lichtbogenerkennung u. a. sehr stark von der Zugkraft der Schlinge auf den Polypenstiel abhängig ist. Durch einen höheren mechanischen Zug bei einer fest angelegten Schlinge entstehen Lichtbögen, die deutlich verzögert gegenüber Lichtbögen bei lose nachgeführter Schlinge erkannt werden würden.

Die der Strommonitoreinrichtung 16 zugeordnete Auswerteeinrichtung 22 erfasst den Stromabfall über die definierte Zeitspanne bzw. den Schwellenwert durch Berechnen des Mittelwertes über eine definierte Anzahl jeweils zuletzt eingelesener Messwerte. Es wird also idealerweise eine permanente Erfassung von Amplitudenwerten des HF-Stromes und eine permanente Mittelwertbildung durchgeführt, um ein Rauschen des Signals zu erkennen und die Wahrscheinlichkeit einer fehlerhaften Abschaltung des HF-Generators 11, d. h. eine Unterbrechung des HF-Stromkreises durch die Strommonitoreinrichtung 16 aufgrund einer falschen Interpretation eines Kurvenverlaufs zu verringern. Die Mittelwertbildung soll vorzugsweise erst zu einem vorgegebenen Zeitpunkt nach Beginn der Messungen erfolgen, so dass sich ein erkennbarer Kurvenverlauf abzeichnet.

Mittels der zweiten Zeitgebereinrichtung 25 lässt sich der Schneidmodus nach dem Beginn der Schneidphase für eine definierte Zeitspanne aktiv halten. Damit kann der Schneidmodus selbsttätig für eine gewünschte Zeitspanne aufrechterhalten werden, ohne dass der Chirurg in den Ablauf eingreifen muss. Die Zeitspanne kann z. B. bereits vor dem Eingriff festgelegt werden.

Die der Lichtbogenmonitoreinrichtung 17 zugeordnete Detektionseinrichtung 23 ist beispielsweise derart ausgelegt, dass sie höhere harmonische Frequenzen und/oder nicht-harmonische Frequenzen des HF-Stromes als eine für den Lichtbogen charakteristische Frequenz detektiert. Durch das Erfassen dieser Frequenzen wird auf einfachste Art die Erkennung eines Lichtbogens bewerkstelligt.

Die dritte Zeitgebereinrichtung 26 ist der Lichtbogenmonitoreinrichtung 17 zugeordnet und ermöglicht auch nach erkanntem Lichtbogen die Ausdehnung der Schneidphase für einen gewünschten Zeitraum.

Wie in Fig. 1 gezeigt, ist sowohl das erste Abschaltsignal c als auch das zweite Abschaltsignal d an die erste Signalverarbeitungseinrichtung 18 über Steuerleitungen C', D' zuführbar. Die erste Signalverarbeitungseinrichtung 18 ist derart ausgebildet, dass das jeweilige Abschaltsignal c oder d als erneutes Einschaltsignal b an den HF-Generator 11 über eine Steuerleitung B übermittelbar ist, so dass dieser nach dem Abschalten oder dem Unterbrechen des HF-Stromkreises erneut einschaltet bzw. den Stromkreis schließt. Dies ist insbesondere bei einem fraktionierten Schneiden, d. h. innerhalb eines intermittierenden Schneidmodus von Vorteil, bei dem erst durch die Abfolge mehrerer Schneidimpulse eine vollständige Gewebedurchtrennung erreicht wird. Damit kann sich der Operateur auf den Eingriff konzentrieren, während die Steuerung der Schneidphase selbsttätig abläuft.

Ist der ersten Signalverarbeitungseinrichtung 18, wie in diesem Ausführungsbeispiel vorgesehen, die erste Zeitgebereinrichtung 24 zugeordnet, so steuert die erste Signalverarbeitungseinrichtung 18 den HF-Generator 11 erst nach einer definierten Zeitspanne an. Damit können beliebig lange Pausenintervalle zwischen den einzelnen Schneidimpulsen vorgesehen werden, um beispielsweise wiederholt eine Abkühlung des Operationsgebietes zu gewährleisten.

Das erste Abschaltsignal c und das zweite Abschaltsignal d sind ebenfalls an die zweite Signalverarbeitungseinrichtung 19 über Steuerleitungen C", D" zuführbar, wobei die zweite Signalverarbeitungseinrichtung 19 mittels des ersten Abschaltsignals c oder des zweiten Abschaltsignals d die optische und/oder akustische Anzeige 21 derart ansteuert, dass das Abschalten des HF-Generators 11 bzw. die Unterbrechung des HF-Stromes aufgrund des ersten Abschaltsignals c oder des zweiten Abschaltsignals d zur Benutzerführung angezeigt wird. Mittels der Anzeige 21 ist es möglich, während des weiteren Eingriffs einen bisher aufgezeichneten Schneidverlauf nachzuvollziehen und damit sowohl die Gewebestrukturen zu beurteilen als auch die weitere Handhabung des elektrochirurgischen Instruments darauf abzustimmen.

Vorzugsweise werden über die Speichereinrichtung 20 die jeweiligen Abschaltsignale c, d abgespeichert, um diese für eine spätere Auswertung über die Anzeige 21 oder auch über einen Ausdruck zur Verfügung zu stellen. Die Aufzeichnungen dienen dazu, daraus ,resultierende Erfahrungen für weitere Eingriffe nutzbar zu machen.

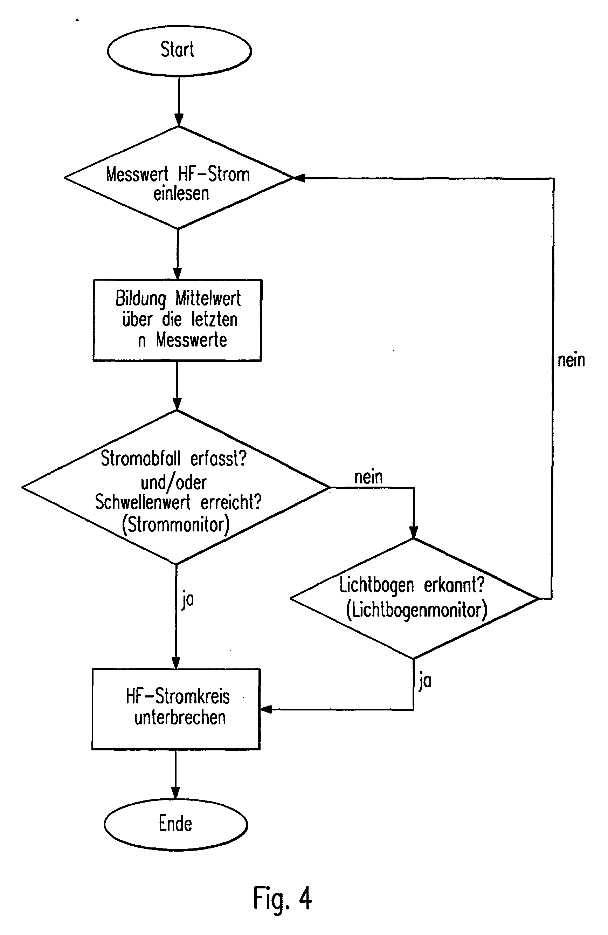

Fig. 4 zeigt ein Flussdiagramm, das eine Arbeitsweise des HF-Chirurgiegerätes 10 gemäß Fig. 1 zeigt, wobei nur wesentliche Komponenten, also die Strommonitoreinrichtung 16 und die Lichtbogenmonitoreinrichtung 17 des HF-Chirurgiegerätes 10 einbezogen sind. Das Flussdiagramm ist sehr verkürzt dargestellt, weil auf die Charakteristik der Entweder-Oder-Beziehung zwischen den Monitoreinrichtungen abgestellt ist.

Der Vorgang beginnt mit dem Einlesen von Messwerten des HF-Stromes, wobei über die definierte Anzahl der zuletzt eingelesenen Messwerte die Mittelwertbildung durchgeführt wird, um entweder die abfallende Charakteristik des HF-Strom-Mittelwerts über die definierte Zeitspanne als erstes Abschaltkriterium oder das Erreichen des den Gewebezustand definierenden Schwellenwerts als erstes Abschaltkriterium zu erfassen. Bei Erreichen des ersten Abschaltkriteriums wird der HF-Generator 11 derart angesteuert, dass dieser den HF-Stromkreis unterbricht, d. h. im einfachsten Fall abschaltet. Wird das Abschaltkriterium nicht erreicht, erfolgt die Überprüfung der Lichtbogenerkennung durch die Lichtbogenmonitoreinrichtung 17. Bei Erfassen eines Lichtbogens wird der HF-Generator 11 abgeschaltet bzw. unterbricht den HF-Stromkreis. Fällt die Lichtbogenerkennung negativ aus, wird erneut ein Messwert des HF-Stromes eingelesen und der Vorgang beginnt erneut.

Das Unterbrechen des HF-Stromkreises kann also, wie bereits mehrfach beschrieben, durch das Abschalten des HF-Generators erfolgen, oder aber der Stromkreis wird anderweitig, z. B. mittels eines durch die Steuerungseinrichtung zu betätigenden Schalters unterbrochen.

An dieser Stelle sei darauf hingewiesen, dass alle oben beschriebenen Teile für sich alleine gesehen und in jeder Kombination, insbesondere die in den Zeichnungen dargestellten Details als erfindungswesentlich beansprucht werden. Abänderungen hiervon sind dem Fachmann geläufig.

### Bezugszeichenliste

- 10: HF-Chirurgiegerät
- 11: HF-Generator
- 12: Eingangsanschluss
- 13: Erster Ausgangsanschluss
- 14: Zweiter Ausgangsanschluss
- 15: Steuerungseinrichtung
- 16: Strommonitoreinrichtung
- 17: Lichtbogenmonitoreinrichtung
- 18: Erste Signalverarbeitungseinrichtung
- 19: Zweite Signalverarbeitungseinrichtung
- 20: Speichereinrichtung
- 21: Anzeige
- 22: Auswerteeinrichtung
- 23: Detektionseinrichtung
- 24: Erste Zeitgebereinrichtung
- 25: Zweite Zeitgebereinrichtung
- 26: Dritte Zeitgebereinrichtung
- 40: Bipolares elektrochirurgisches Instrument
- 50a: Monopolares elektrochirurgisches Instrument
- 50b: Neutralelektrode
- 60: Körperabschnitt eines Patienten

- a: Einschaltsignal
- b: Einschaltsignal
- c: Erstes Abschaltsignal
- d: Zweites Abschaltsignal
- B: Steuerleitung
- C, C', C": Steuerleitung
- D, D', D": Steuerleitung

- I_{HF}: HF-Stromstärke
- t, t': Zeit, Zeitpunkt

## Patentansprüche

1. HF-Chirurgiegerät zum Behandeln, insbesondere zum Schneiden und Koagulieren biologischen Gewebes mittels eines HF-Stromes, umfassend
- einen HF-Generator (11) zum Zuführen eines HF-Stromes zu einer Schneidelektrode (50a),
- mindestens eine Steuerungseinrichtung (15) zum Unterbrechen eines HF-Stromkreises,
wobei
die Steuerungseinrichtung (15) eine Lichtbogenmonitoreinrichtung (17) zur Erkennung eines Lichtbogens und eine Strommonitoreinrichtung (16) zur Erfassung des HF-Stromes umfasst, wobei
- die Strommonitoreinrichtung (16) derart ausgebildet ist, dass sie die Amplitude des HF-Stromes erfasst und ein erstes Abschaltsignal (c) dann erzeugt, wenn als ein erstes Abschaltkriterium der HF-Strom über eine definierte Zeitspanne abfällt und/oder der HF-Strom einen Zustand des behandelten Gewebes kennzeichnenden Schwellenwert unterschreitet,
- die Lichtbogenmonitoreinrichtung (17) derart ausgebildet ist, dass sie ein zweites Abschaltsignal (d) dann erzeugt, wenn als ein zweites Abschaltkriterium ein Lichtbogen zwischen der Schneidelektrode (50a) und dem Gewebe erkennbar entsteht,
wobei die Steuerungseinrichtung (15) zur Erfassung einer Schneidwirkung während einer Dampfphase derart ausgebildet ist, dass ein Erreichen des ersten Abschaltkriteriums überprüft wird und bei Nicht-Erreichen des ersten Abschaltkriteriums das zweite Abschaltkriterium überprüft wird, so dass auf das erste Abschaltsignal (c) oder das zweite Abschaltsignal (d) hin der HF-Stromkreis unterbrochen wird,
**dadurch gekennzeichnet, dass**
der Strommonitoreinrichtung (16) eine zweite Zeitgebereinrichtung (25) zugeordnet ist, die einen Schneidmodus nach dem Erzeugen des ersten Abschaltsignals (c) für eine definierte Zeitspanne aktiv hält, und dass der Lichtbogenmonitoreinrichtung (17) eine dritte Zeitgebereinrichtung (26) zugeordnet ist, die den Schneidmodus nach dem Erkennen des Lichtbogens für eine definierte Zeitspanne aktiv hält.

2. HF-Chirurgiegerät nach Anspruch 1,
**dadurch gekennzeichnet, dass**
die Steuerungseinrichtung (15) das erste Abschaltsignal (c) oder das zweite Abschaltsignal (d) an den HF-Generator (11) übermittelt, so dass dieser abschaltet und den HF-Stromkreis unterbricht.

3. HF-Chirurgiegerät nach Anspruch 1 oder 2,
**dadurch gekennzeichnet, dass**
mindestens eine erste Signalverarbeitungseinrichtung (18) vorgesehen ist, an die das erste Abschaltsignal (c) oder das zweite Abschaltsignal (d) zuführbar ist, wobei die erste Signalverarbeitungseinrichtung (18) das jeweilige Abschaltsignal (c, d) als Einschaltsignal (b) an den HF-Generator (11) übermittelt, so dass dieser einschaltet und der HF-Stromkreis geschlossen wird.

4. HF-Chirurgiegerät nach einem der vorhergehenden Ansprüche, insbesondere nach Anspruch 3,
**dadurch gekennzeichnet, dass**
der mindestens ersten Signalverarbeitungseinrichtung (18) mindestens eine erste Zeitgebereinrichtung (24) zugeordnet ist, so dass die erste Signalverarbeitungseinrichtung (18) den HF-Generator (11) derart ansteuert, dass das Einschalten des HF-Generators (11) nach einer definierten Zeitspanne erfolgt.

5. HF-Chirurgiegerät nach einem der vorhergehenden Ansprüche,
**dadurch gekennzeichnet, dass**
der Strommonitoreinrichtung (16) eine Auswerteeinrichtung (22) zugeordnet ist, die die Amplitude des HF-Stromes durch Berechnen des Mittelwertes über eine definierte Anzahl jeweils zuletzt eingelesener Messwerte erfasst.

6. HF-Chirurgiegerät nach einem der vorhergehenden Ansprüche,
**dadurch gekennzeichnet, dass**
der Lichtbogenmonitoreinrichtung (17) eine Detektionseinrichtung (23) zugeordnet ist, die höhere harmonische Frequenzen und/oder nicht-harmonische Frequenzen des HF-Stromes als charakteristische Frequenz für ein Vorhandensein des Lichtbogens detektiert.

7. HF-Chirurgiegerät nach einem der vorhergehenden Ansprüche,
**dadurch gekennzeichnet, dass**
mindestens eine zweite Signalverarbeitungseinrichtung (19) vorgesehen ist, an die das erste Abschaltsignal (c) oder das zweite Abschaltsignal (d) zuführbar ist, wobei die zweite Signalverarbeitungseinrichtung (19) mittels des ersten Abschaltsignals (c) oder des zweiten Abschaltsignals (d) eine optische und/oder akustische Anzeige (21) derart ansteuert, dass die Unterbrechung des HF-Stromkreises aufgrund des ersten Abschaltsignals (c) oder des zweiten Abschaltsignals (d) zur Benutzerführung angezeigt wird.

8. HF-Chirurgiegerät nach einem der vorhergehenden Ansprüche,
**dadurch gekennzeichnet, dass**
mindestens eine Speichereinrichtung (20) vorgesehen ist, die die jeweils generierten Abschaltsignale (c, d) innerhalb eines Eingriffs zum späteren und/oder gleichzeitigen Anzeigen eines Schneidverlaufes speichert.

9. HF-Chirurgiegerät nach einem der vorhergehenden Ansprüche,
**dadurch gekennzeichnet, dass**
die Schneidelektrode als eine Schlingenelektrode (50a) ausgebildet ist.

## Claims

1. Electrosurgical device for treating, more particularly for cutting and coagulating, biological tissue by means of an RF current, comprising
- an RF generator (11) for supplying an RF current to a cutting electrode (50a),
- at least one control apparatus (15) for interrupting an RF circuit,
with
the control apparatus (15) comprising an electric-arc monitoring apparatus (17) for identifying an electric arc and a current monitoring apparatus (16) for detecting the RF current, with
- the current monitoring apparatus (16) being embodied such that it detects the amplitude of the RF current and produces a first switch-off signal (c) if, as a first switch-off criterion, the RF current falls during a defined period of time and/or the RF current drops below a threshold characterizing the state of the treated tissue,
- the electric-arc monitoring apparatus (17) being embodied such that it produces a second switch-off signal (d) if, as a second switch-off criterion, an electric arc is identifiably created between the cutting electrode (50a) and the tissue,
with the control apparatus (15) for detecting a cutting effect during a vapour phase being embodied such that reaching the first switch-off criterion is monitored and, if the first switch-off criterion is not reached, the second switch-off criterion is monitored such that the RF circuit is interrupted following the first switch-off signal (c) or the second switch-off signal (d),
**characterized in that**
a second timer apparatus (25) is associated with the current monitoring apparatus (16), the former keeping a cutting mode active for a defined period of time after the first switch-off signal (c) is produced, and **in that** a third timer apparatus (26) is associated with the electric-arc monitoring apparatus (17), the former keeping the cutting mode active for a defined period of time after the electric arc is identified.

2. Electrosurgical device according to Claim 1,
**characterized in that**
the control apparatus (15) transmits the first switch-off signal (c) or the second switch-off signal (d) to the RF generator (11) such that the latter is switched off and interrupts the RF circuit.

3. Electrosurgical device according to Claim 1 or 2,
**characterized in that**
provision is made for at least one first signal-processing apparatus (18), to which the first switch-off signal (c) or the second switch-off signal (d) can be supplied, with the first signal-processing apparatus (18) transmitting the respective switch-off signal (c, d) as switch-on signal (b) to the RF generator (11) such that the latter is switched on and the RF circuit is closed.

4. Electrosurgical device according to one of the preceding claims, more particularly according to Claim 3,
**characterized in that**
at least a first timer apparatus (24) is associated with the at least first signal-processing apparatus (18) such that the first signal-processing apparatus (18) actuates the RF generator (11) such that the RF generator (11) is switched on after a defined period of time.

5. Electrosurgical device according to one of the preceding claims,
**characterized in that**
an evaluation apparatus (22) is associated with the current monitoring apparatus (16), the former detecting the amplitude of the RF current by calculating the mean value over a defined number of respectively most-recently read measurement values.

6. Electrosurgical device according to one of the preceding claims,
**characterized in that**
a detection apparatus (23) is associated with the electric-arc monitoring apparatus (17), the former detecting higher harmonic frequencies and/or non-harmonic frequencies of the RF current as a characteristic frequency for presence of the electric arc.

7. Electrosurgical device according to one of the preceding claims,
**characterized in that**
provision is made for at least one second signal-processing apparatus (19), to which the first switch-off signal (c) or the second switch-off signal (d) can be supplied, with the second signal-processing apparatus (19) actuating an optical and/or acoustic indicator unit (21) by means of the first switch-off signal (c) or of the second switch-off signal (d) such that the interruption of the RF circuit as a result of the first switch-off signal (c) or of the second switch-off signal (d) is indicated for user guidance.

8. Electrosurgical device according to one of the preceding claims,
**characterized in that**
provision is made for at least one storage apparatus (20), which stores the respectively generated switch-off signals (c, d) within an intervention for later and/or simultaneous indication of a cut progression.

9. Electrosurgical device according to one of the preceding claims,
**characterized in that**
the cutting electrode is embodied as a loop electrode (50a).

## Revendications

1. Instrument chirurgical HF pour traiter, notamment pour couper et coaguler un tissu biologique au moyen d'un courant HF, comprenant
- un générateur HF (11) pour fournir un courant HF à une électrode de coupe (50a),
- au moins un dispositif de commande (15) pour interrompre un circuit électrique HF,
dans lequel
le dispositif de commande (15) comporte un dispositif de surveillance d'arc électrique (17) pour la détection d'un arc électrique et un dispositif de surveillance du courant (16) pour l'enregistrement du courant HF, dans lequel
- le dispositif de surveillance du courant (16) est développé de telle sorte qu'il enregistre l'amplitude du courant HF et qu'il génère un premier signal d'arrêt (c) lorsqu'en tant que premier critère d'arrêt, le courant HF décroit pendant un délai défini et/ou que le courant HF passe au-dessous d'une valeur de seuil caractéristique d'un état du tissus traité,
- le dispositif de surveillance d'arc électrique (17) est développé de telle sorte qu'il génère un deuxième signal d'arrêt (d) lorsqu'en tant que deuxième critère d'arrêt, un arc électrique se produit de manière perceptible entre l'électrode de coupe (50a) et le tissu,
dans lequel le dispositif de commande (15) pour l'enregistrement d'une efficacité de coupe pendant une phase vapeur est développé de manière à contrôler une arrivée du premier critère d'arrêt et lorsque le premier critère d'arrêt n'est pas atteint, à contrôler le deuxième critère d'arrêt, de manière à interrompre le circuit électrique HF suite au premier signal d'arrêt (c) ou au deuxième signal d'arrêt (d).
**caractérisé en ce que**
le dispositif de surveillance du courant (16) comporte un deuxième dispositif d'horloge (25) qui maintient un mode de coupe actif pendant un délai défini après la génération du premier signal d'arrêt (c), et **en ce que** le dispositif de surveillance d'arc électrique (17) comporte un troisième dispositif d'horloge (26) qui maintient le mode de coupe actif pendant un délai défini après la détection de l'arc électrique.

2. Instrument chirurgical HF selon la revendication 1,
**caractérisé en ce que**
le dispositif de commande (15) transmet le premier signal d'arrêt (c) ou le deuxième signal d'arrêt (d) vers le générateur HF (11) de telle sorte que celui-ci s'arrête et interrompt le circuit électrique HF.

3. Instrument chirurgical HF selon la revendication 1 ou 2,
**caractérisé en ce que**
au moins un premier dispositif de traitement de signal (18) est prévu vers lequel on peut acheminer le premier signal d'arrêt (c) ou le deuxième signal d'arrêt (d), le premier dispositif de traitement de signal (18) transmettant le signal d'arrêt respectif (c, d) sous la forme de signal de mise en marche (b) vers le générateur HF (11) de telle sorte que celui-ci se mette en marche et ferme le circuit électrique HF.

4. Instrument chirurgical HF selon une des revendications précédentes, notamment selon la revendication 3,
**caractérisé en ce que**
au moins le premier dispositif de traitement de signal (18) comporte au moins un premier dispositif d'horloge (24), de telle sorte que le premier dispositif de traitement de signal (18) commande le générateur HF (11) de telle sorte que la mise en marche du générateur HF (11) s'effectue après un délai défini.

5. Instrument chirurgical HF selon une des revendications précédentes,
**caractérisé en ce que**
le dispositif de surveillance du courant (16) comporte un dispositif d'analyse (22) qui enregistre l'amplitude du courant HF en calculant la valeur moyenne sur un certain nombre des dernières valeurs de mesure respectivement enregistrées.

6. Instrument chirurgical HF selon une des revendications précédentes,
**caractérisé en ce que**
le dispositif de surveillance d'arc électrique (17) comporte un dispositif de détection (23) qui détecte des fréquences harmoniques plus élevées et/ou des fréquences non harmoniques du courant HF en tant que fréquence caractéristique de la présence de l'arc électrique.

7. Instrument chirurgical HF selon une des revendications précédentes,
**caractérisé en ce que**
au moins un deuxième dispositif de traitement de signal (19) est prévu vers lequel on peut acheminer le premier signal d'arrêt (c) ou le deuxième signal d'arrêt (d), le deuxième dispositif de traitement de signal (19) commandant au moyen du premier signal d'arrêt (c) ou du deuxième signal d'arrêt (d), une indication optique et/ou acoustique (21) de manière à indiquer l'interruption du circuit électrique HF en raison du premier signal d'arrêt (c) ou le deuxième signal d'arrêt (d) pour le guidage de l'utilisateur.

8. Instrument chirurgical HF selon une des revendications précédentes,
**caractérisé en ce que**
au moins un dispositif de mémorisation (20) est prévu qui mémorise les signaux d'arrêt (c, d) générés respectivement lors d'une intervention pour une indication ultérieure et/ou simultanée à l'opération de coupe.

9. Instrument chirurgical HF selon une des revendications précédentes,
**caractérisé en ce que**
l'électrode de coupe est développée sous la forme d'une électrode en boucle (50a).
